# EUROPEAN PATENT SPECIFICATION

(11) **EP 1 861 465 B1**
(45) Date of publication and mention of the grant of the patent: **13.10.2010**
(21) Application number: 06723325.4
(22) Date of filing: 09.03.2006
(51) Int. Cl.: C09C 1/36, C09C 3/12, A61Q 17/04, A61K 8/11, A61K 8/29, C08G 77/16, C07D 249/18, C07D 249/20

(54) **CHROMOPHORE COATED METAL OXIDE PARTICLES**
CHROMOPHORBESCHICHTETE METALLOXIDPARTIKEL
PARTICULES D'OXYDE METALLIQUE REVETUES D'UN CHROMOPHORE

(30) Priority: 23.03.2005 EP 05006432
(43) Date of publication of application: 05.12.2007
(73) Proprietor: DSM IP Assets B.V., 6411 TE Heerlen (NL)
(72) Inventor: SCHEHLMANN, Volker, 79650 Schopfheim (DE); BERG-SCHULTZ, Katja, CH-4303 Kaiseraugst (CH)
(74) Representative: Best, Michael
(86) International application number: PCT/EP2006/002185
(87) International publication number: WO 2006/099952

(56) References cited:
- EP-A- 0 478 284
- WO-A-2005/053631
- DE-A1- 10 333 029
- X. ZHANG, S. DU, Y. CHEN, L. ZHANG, Y. CAO, X. CHAI, Y. BAI, L. XIAO, T. LI: "Preparation of covalently modified organic-inorganic composite nanoparticles and their interfacial electron transfer researches" THIN SOLID FILMS, vol. 327-329, 1998, pages 563-567, XP002401516
- SPANGE S ET AL: "A one-pot synthesis of chromophoric silicate-based xerogels" ANGEWANDTE CHEMIE, WILEY-VCH, WEINHEIM, DE, vol. 41, no. 10, 2002, pages 1729-1732, XP002327219 ISSN: 1433-7851

## Description

The present invention relates to novel UV filters, a process for the preparation and their use especially in formulations for the protection of human skin or hair against harmful effects of sunlight. The novel UV filters are metal oxide particles which are coated with a mixture of crosslinkable monomers comprising at least one type of crosslinkable chromophores.

There is a constantly increasing need for sunscreen protection agents in a population that is exposed to an increasing amount of damaging sunlight. Repetitive sun exposure can result in skin changes known as photoaged skin. The clinical changes that are seen in photoaged skin differ from those of normally aged skin in sunlight protected sites of the body. Among the damaging results of intensive sun exposure of the skin there is increased wrinkling, elastosis, pigmentary changes, precancerous and cancerous skin lesions.

Many sun screening chemicals have been developed in the past protecting against the harmful effect of UV-A (320 to 400 nm) or UV-B (290-320 nm) wavelength and even shorter wavelength (UV-C). Very recently a new class of sun screening chemicals, the broadband UV-filters have been developed which shield the skin from UV-A and UV-B radiation. These chemicals are usually incorporated either alone or in combination with each other into cosmetic or pharmaceutical preparations which are widely known and used.

Most UV filters used in cosmetic or dermatological compositions are monomeric compounds, and thus there is the inherent risk that such compounds can penetrate the skin barrier, which is highly undesirable. UV filters on the basis of polysiloxanes which may be either linear or cyclic are described e.g. in WO 93/04665, WO 94/06404, EP-A 538 431, EP-A 392 883 and EP-A 358 584. With these polysiloxanes the risk of skin penetration is lower, but it is sometimes difficult to incorporate the polysiloxanes in cosmetic or dermatological compositions due to incompatibility problems which differ depending on the UV-active chromophores which are covalently bonded to the polysiloxanes.

EP-A 1 205 178 and EP-A 1 205 177 suggest to reduce the risk of penetration of active ingredients such as UV filters into the skin and possible damage of the skin or allergies caused thereby by immobilizing the active ingredients or UV filters present in a dermatological or cosmetic composition. These documents disclose a conjugate which comprises an anorganic pigment and an active ingredient on the basis of an organic compound which is covalently bonded via a spacer group to the anorganic pigment. The conjugate disclosed in these documents thus contains an anorganic pigment and to the surface of this pigment chromophores are chemically bonded.

Apart from carbon-based chromophores metal oxides such as titanium dioxide or zinc oxide are widely used in sunscreening agents. This action is substantially based on the reflection, scattering and absorption of harmful UV radiation and is substantially dependent upon the primary particle size of the metal oxide.

However, incorporation of metal oxide in sunscreen compositions can cause significant problems, in particular because many metal oxides such as titanium dioxide or zinc oxide tend to agglomerate in many finished formulations leading to a loss in their efficacy as sunscreening agent and resulting in an unacceptable aesthetic appearance due to a so-called 'whitening effect' when applied to the skin. Furthermore, the metal oxides such as titanium dioxide or zinc oxide show formulation incompatibilities when used in combination with other usual ingredients of cosmetic or dermatological formulations. Especially unwanted are incompatibilities with other UV-sunscreens such as dibenzoylmethane derivatives (e.g. 4,4'-Methoxy-tert.butyldibenzoylmethane) resulting amongst others in a coloration of the formulation which is highly undesirable. Additionally, untreated metal oxides are known to have photocatalytic activity through which reactions are triggered which may lead to changes of components of sunscreening agents or cause skin irritations.

Various coatings have been proposed for modifying the surface of the metal oxide in order to ease the incorporation into the formulation, to avoid the formulation incompatibilities and to reduce the photocatalytic activity such as e.g. simethicone, methicone and triethoxy caprylylsilane and silicium dioxide e.g. described in EP-A 1 281 388 and DE-A 103 33 029. In particular in view of their photocatalytic activity it has been suggested to coat the metal oxide particles with an inorganic coating which is not degenerated by a photocatalytic reaction. Coatings on the basis of calcinated alumina and of other oxides are known. EP-A 988 853, EP-A 1 284 277, and US-A 5,562,897 disclose silica coated metal oxide powders.

While these coatings solve some problems associated with the use of metal oxide particles as UV absorbing or scattering particles in sunscreen compositions, there is still need for improved coated metal oxide particles, in particular for UV absorbing and/or scattering particles which allow a wide variability regarding the range of UV light which they absorb or scatter while nevertheless avoid the manifold problems occurring after incorporation of those UV absorbing particles into cosmetic or dermatological compositions.

There is a need to provide UV filters which do not show the shortcomings of the prior art UV filters, They should have excellent UV filter activity, being easily accessible and compatible with the other usual ingredients of cosmetic and dermatological compositions and with other UV-sunscreens (or UV-filter, both terms are used interchangeably) e.g. such as dibenzoylmethane derivatives.

For solving these problems the present invention for the first time provides novel UV filter particles which combine both the advantages of coated metal oxide particles and the advantages of chromophores on carbon basis which have excellent UV filter activity over a broad wavelength independent of the particle size. With these novel UV filter particles sunscreen formulations with high SPF values can be generated while avoiding the disadvantages of the prior art metal oxide particles and the prior art chromophores on carbon basis. Surprisingly, it was also found that the metal oxides of this invention additionally stabilize dibenzoylmethane derivatives such as 4,4'-Methoxy-tert.butyldibenzoylmethane against degradation upon irradiation which are well known to have a limited photostability.

The present invention therefore provides a process for producing novel coated metal oxide particles, wherein metal oxide particles are coated with at least one type of crosslinkable chromophore with UV-A and/or UV-B and/or UV-C filter and/or broadband activity which is a monomer of the formula M(R)ₙ(P)ₘ(Q)_{q}, wherein M is selected from the group consisting of silicon, titanium, zinc, aluminium and zirkonium, R is a hydrolysable group, P is a chromophore with UV-A, UV-B and/or UV-C and/or broadband filter activity, Q is a non-hydrolysable group, n is 2 or 3, m is 1 or 2 and q is 0 or 1, wherein n+m+q=4 and optionally at least one type of crosslinkable monomer which does not have UV-A and/or UV-B and/or UV-C and/or broadband filter activity. The present invention also provides the novel coated metal oxide particles obtainable by this process and cosmetic or dermatological compositions comprising those coated metal oxide particles and the use of the coated metal oxide particles for producing cosmetic or dermatological compositions especially sunscreen compositions. Additionally, the present invention provides a method for the stabilization of dibenzoylmethane derivatives against degradation upon irradiation.

Preferably in the process for producing the coated metal oxide particles according to the present invention both crosslinkable chromophores with UV filter activity, either one or mixtures of crosslinkable chromophores with UV filter activity having UV-A and/or UV-B and/or UV-C and/or broadband filter activity and crosslinkable monomers which do not have UV filter activity are present so that the final coating of the metal oxide particles is composed of units from crosslinkable monomers with no UV filter activity and of units from crosslinkable chromophores with UV filter activity.

The term "UV filter activity" as used herein encompasses UV-A, UV-B, UV-C and broadband filter activity, preferably UV-A, UV-B and broadband filter activity. If the term "UV filter" or "UV filter activity" is used without further explanation, it refers to compounds or compositions or moieties which have either UV-A or UV-B or UV-C or broadband filter activity as well as to compounds or compositions or moieties which have both UV-A and UV-B filter activity and to compounds or compositions or moieties which have UV-A, UV-B and UV-C filter activity and to compounds or compositions or moieties which have both UV-B and UV-C filter activity. Preferably, the term "UV filter" or "UV filter activity" is used for compounds, compositions or moieties which have UV-A, UV-B or broadband filter activity (UV-A- and UV-B-filter activity)

The crosslinkable chromophores with UV filter activity according to the present invention are chromophores of the formula M(R)ₙ(P)ₘ(Q)_{q},
wherein
**M** is selected from the group consisting of silicon, titanium, zinc, aluminum and zirconium, preferably, M is silicon
**R** is a hydrolysable substituent with preferably not more than 10 carbon atoms (such as an alkoxide, aryloxide, carboxylic ester, acyloxy group, diketonato group, hydrolysable aza group or chlorine), more preferably an alkoxide or an acyloxy group with not more than 10 carbon atoms, in particular a C₁-C₆ alkoxide such as a methoxide or an ethoxide
**Q** is a non-hydrolysable group, preferably a hydrocarbon group with not more than 10 carbon atoms, such as a C₁-C₈ alkyl group, e.g. a methyl or ethyl or octyl group,
**n** is an integer of 2 or 3, preferably 3,
**m** is an integer of 1 or 2, and
**q** is an integer of 0 or 1 and
**n+m+q=4.**

**P** is the moiety of the molecules which provides the UV filter activity and preferably has a general formula **A-(B)_{b}(C)_{c}(D)_{d}(E)ₑ-** which is chemically bonded to M wherein
**A** is a chromophore with UV-A and/or UV-B and/ or broadband filter activity and - **(B)_{b}(C)_{c}(D)_{d}(E)ₑ-** is a spacer group in which
**B** is a linear or branched alkylene group with up to 20, preferably 1-12, most preferred 3-12 carbon atoms
**C** is 0, S or NH
**D** is a CONH- group
**E** is a linear or branched alkylene or alkenylene group with up to 20, preferably 1-12, most preferred 3-12 carbon atoms and
**b** is 0 or 1,
**c** is 0 or 1,
**d** is 0 or 1 and
**e** is 0 or 1.

Preferably, M is silicon, R has not more than 10 carbon atoms and is an alkoxide, an aryloxide or an acyloxy group, more preferably an alkoxide or an acyloxy group with not more than 10 carbon atoms, in particular a C₁-C₆ alkoxide such as a methoxide or an ethoxide, n is an integer of 2 or 3, in particular of 3, P is a residue A with UV-A and/or UV-B and/ or UV-C filter activity which is chemically bonded to M usually by a spacer group (P is the combination of A and the spacer group) and m is 1. The spacer group has preferably 1 to 10 carbon atoms and optionally 1 to 3 hetero atoms such as nitrogen or oxygen atoms and is preferably of the formula -(B)_{b}(C)_{c}(D)_{d}(E)ₑ- as defined above.

The preparation of crosslinkable chromophores with UV filter activity is within the knowledge of the skilled person, and some preferred examples are generally disclosed in the following:

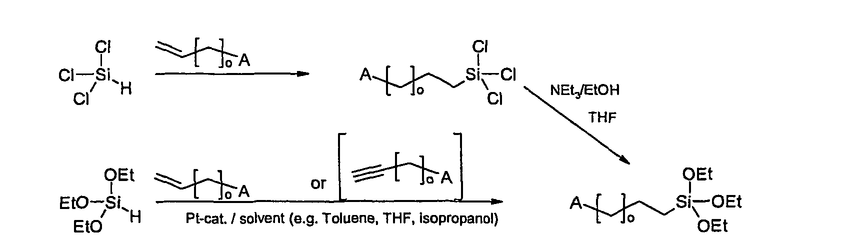

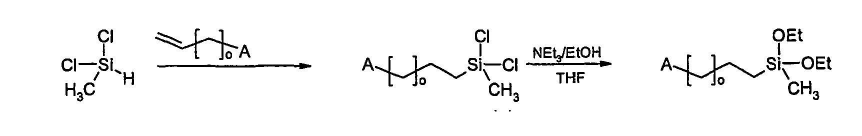

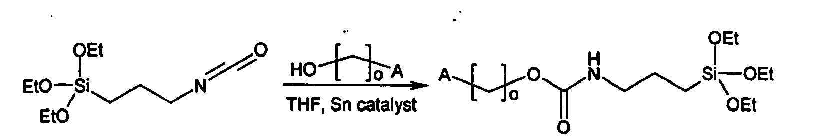

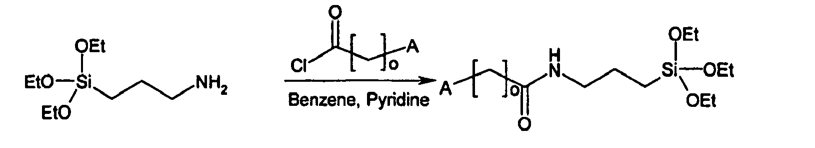

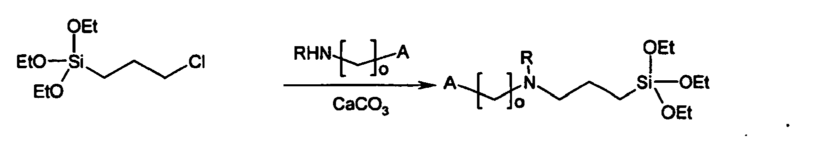

In the above scheme residue A is the part of the residue P which provides the UV filter activity, and residue P usually consists of a residue A and optionally a spacer group which is used for coupling the residue A to residue M, in particular to the silicon, index o is 0 to 6, preferably 0, 1, 2 or 3.

In the above scheme preferred embodiments of the invention have been exemplified in which residue M is silicon, and all residues R are chlorine or an ethoxide. Embodiments in which n is 3 and m is 1 are particularly preferred. Of course, the processes are also applicable for other embodiments, in particular for other alkoxides such as methoxide.

The residues A are preferably known UV light absorbers, and these known UV light absorbers are modified preferably by functional silanes such as methyldichlorosilane, trichlorosilane, triethoxysilane, 3-(isocyanatopropyl)triethoxysilane, or 3-(chloropropyl)-triethoxysilane or 3-(aminopropyl)-triethoxysilane without being limited thereto e.g. by
- Hydrosilylation between an allyl or propargyl-terminated molecule A and triethoxysilane, methyldichlorosilane (MDCIS) or the trichlorosilane (TCIS). This coupling reaction results in a C-Si bond;
- Addition of an hydroxyl or amino -terminated molecule A on the 3-(isocyanatopropyl)triethoxysilane (ICPTEOS) resulting in a carbamate or urethane link;
- Amidation of an acyl chloride-terminated molecule M using the 3-(aminopropyl)-triethoxysilane (APTEOS).
- Nucleophilic substitution of a chlorine such as in 3-(chloropropyl)-triethoxysilane (CPTEOS) with a primary or secondary amine whereas the amine can be member of a heterocyclic ring.

The UV-light absorbing groups A covalently bonded to the coating of the metal oxide particles comprise all groups which absorb light in the range of wavelengths 400 - 320 nm (UVA) and/or 320 - 290 (UVB) or of even shorter wavelengths (UVC) and which are or can be used as chemical UV filters. These groups are, e.g., residues of compounds belonging to the groups of acrylates, p-aminobenzoates, camphor derivatives (such as of benzylidene camphor type), cinnamates, benzophenones, esters of benzalmalonic acid, esters of 2-(4-ethoxy anilinomethylene)propandioic, imidazole derivatives, salicylates, triazone derivatives, triazol derivatives, dibenzoylmethanes, amino substituted hydroxybenzophenones, phenylbenzimidazoles, anthranilates, phenyl-benzoxazoles, 1,4-dihydropyranes and others representing state of the art and known to those skilled in the art to be highly active.

Examples for acrylates include 2-ethylhexyl 2-cyano-3,3-diphenylacrylate (octocrylene, PARSOL® 340) and ethyl 2-cyano-3,3-diphenylacrylate;

Examples for p-aminobenzoates include 4-amino benzoic acid, 4-aminobenzoic acid-2,3-dihydroxypropylester, 4-(bis(2-hydroxypropyl)amino)benzoic acid ethyl ester, 4-(dimethylamino)benzoic acid-2-ethylhexylester (e.g. Eusolex® 6007) and ethoxylated 4-aminobenzoic acid ethyl ester (e.g. Uvinul® P25).

Examples for camphor derivatives include 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor and therephthalidene dicamphor sulfonic acid;

Examples for cinnamates include octyl methoxycinnamate (PARSOL® MCX), ethoxyethyl methoxycinnamate, diethanolamine methoxycinnamate (PARSOL® Hydro) and isoamyl methoxycinnamate.

Examples for benzophenones include benzophenone-3, benzophenone-4, 2,2', 4, 4' tetrahydroxy-benzophenone and 2,2'Dihydroxy-4,4'dimethoxybenzophenone;

Examples for esters of benzalmalonic acid include di(2-ethylhexyl) 4-methoxybenzalmalonate

Examples for esters of 2-(4-ethoxy anilinomethylene)propandioic acid include 2-(4-ethoxy anilinomethylene)propandioic acid diethyl ester as described in the European Patent Publication EP 0895 776

Examples for imidazole derivatives include 2-phenyl benzimidazole sulfonic acid and its salts (PARSOL®HS). Salts of 2-phenyl benzimidazole sulfonic acid are e.g. alkali salts such as sodium- or potassium salts, ammonium salts, morpholine salts, salts of primary, sec. and tert. amines like monoethanolamine salts and diethanolamine salts.

Examples for salicylate derivatives include isopropylbenzyl salicylate, benzyl salicylate, butyl salicylate, octyl salicylate (NEO HELIOPAN OS), isooctyl salicylate or homomenthyl salicylate (homosalate, HELIOPAN);

Examples for triazone derivatives include octyl triazone (UVINUL T-150), dioctyl butamido triazone (UVASORB HEB).

Examples for triazol derivatives include benzotriazoles such as 2-(2-hydroxy-5-methylphanyl)benzotriazol, 2,2'-methylene-bis-(6-(2H-benzotriazole-2-yl)-4-(1,1,3,3,-tetramethylbutyl)-phenol (TINOSORB M) as well as triazols described in EP-A-893119

Examples for dibenzoylmethane derivatives include compounds such as 4-tert. butyl-4'-methoxydibenzoyl-methane (PARSOL^{®} 1789), dimethoxydibenzoylmethane and isopropyldibenzoylmethane;

Examples for Amino substituted hydroxybenzophenones include compounds such as 2-(4-Diethylamino-2-hydroxy-benzoyl)-benzoic acid hexyl ester as described in the European Patent Publication EP 1046391

Preferred residues A are
Benzophenone derivatives such as 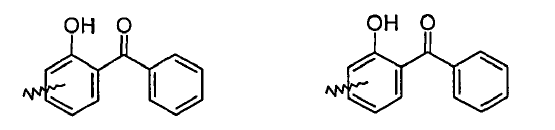 p-Aminobenzoic acid derivatives such as 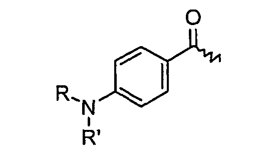 Benzoxazole derivatives such as 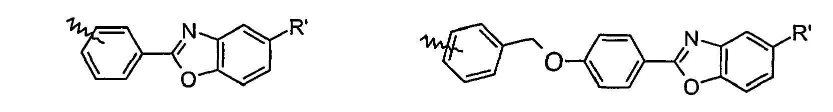 Camphor derivatives such as 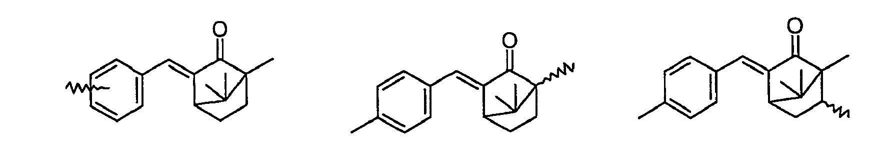 Cinnamic acid or benzalmalonate derivatives such as 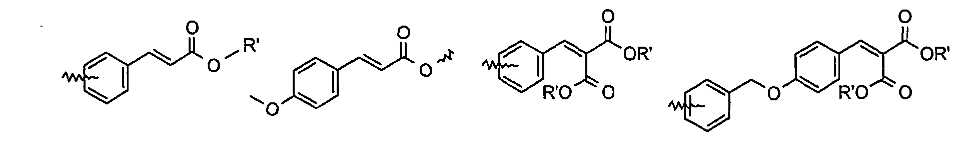 Benzimidazole derivatives such as 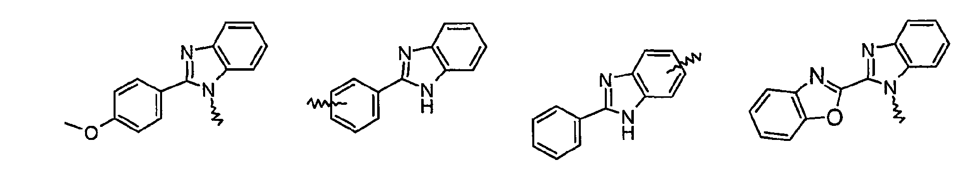 Octocrylene derivatives such as 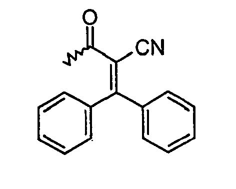 Benzotriazol derivatives such as 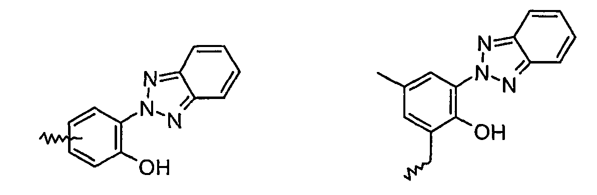 Dihydropyridine Derivatives such as 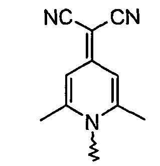 tert-Butyldibenzoylmethane derivatives such as 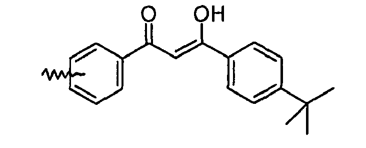 wherein R' is hydrogen, hydroxy, straight or branched chain C₁₋₂₀-alkyl, -alkoxy or C₂₋₂₀-alkenyl.

Preferably, the crosslinkable chromophore with UV filter activity is obtainable by reacting a silane molecule, e.g. of the formula Si(R)ᵣ(Q)_{q}S, wherein R is a hydrolysable group as defined above, in particular an alkoxy group or an acyloxy group with 10 carbon atoms or less, Q is a non-hydrolysable group as defined above, in particular a C₁-C₆ alkyl group, e.g. methyl, S is a reactive group which can react with the chromophore, in particular a hydrogen atom, a group -(CH₂)ₒ-NCO, a group -(CH₂)ₒ-Cl or a group -(CH₂)ₒ-NH₂, r is 2 or 3, q is 0 or 1 and o is 1 to 6, such as 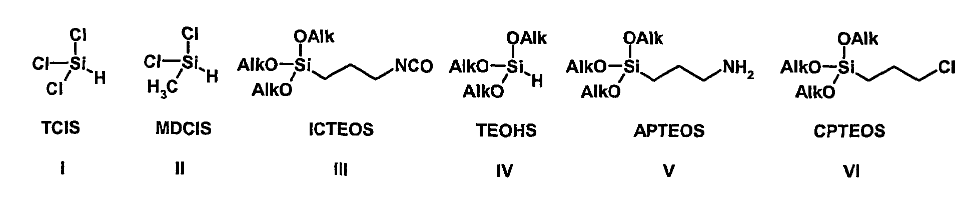 wherein Alk is a C₁-C₆ alkyl group, preferably methyl or ethyl,
with a chromophore selected e.g. from 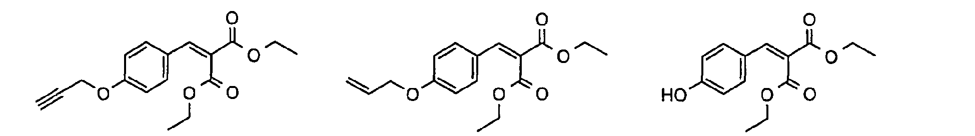 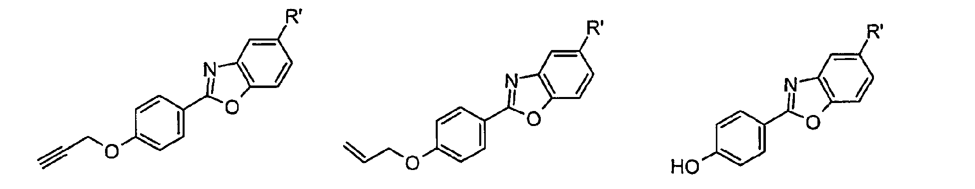 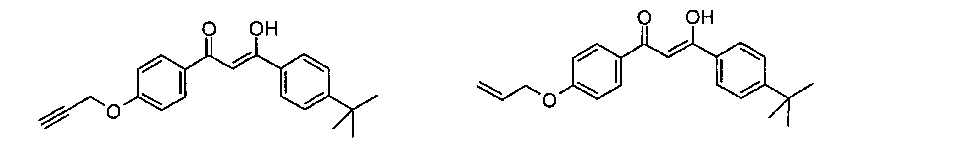 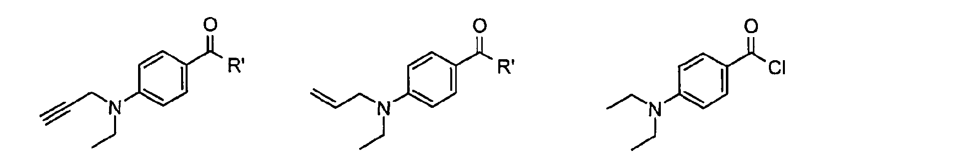 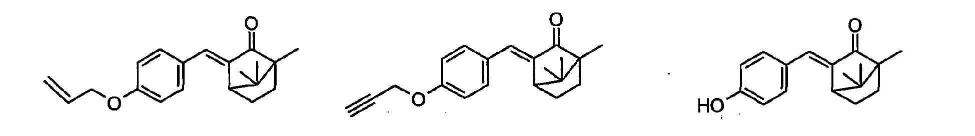 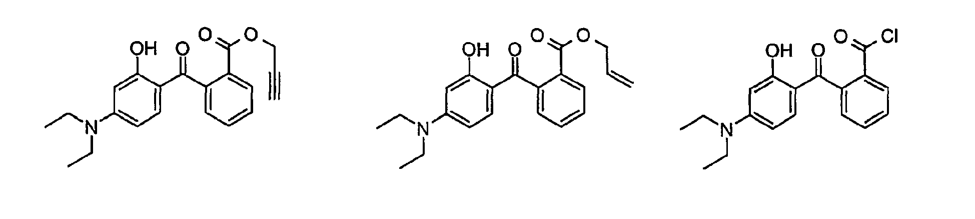 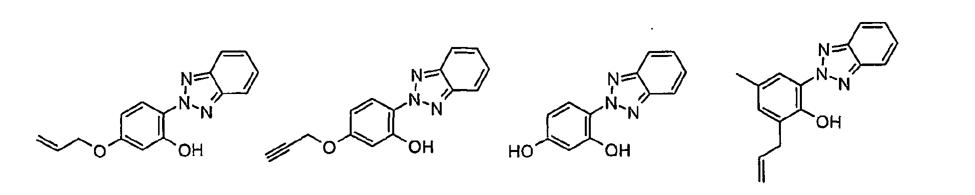 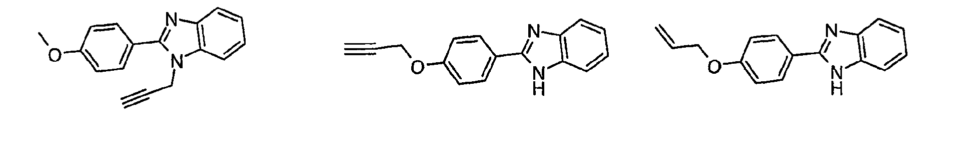 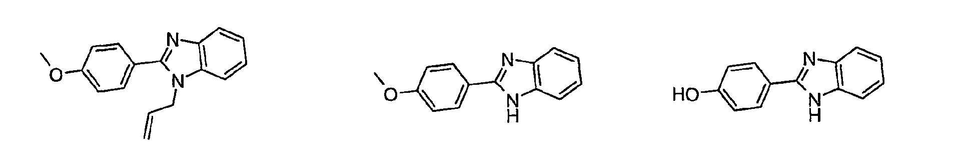 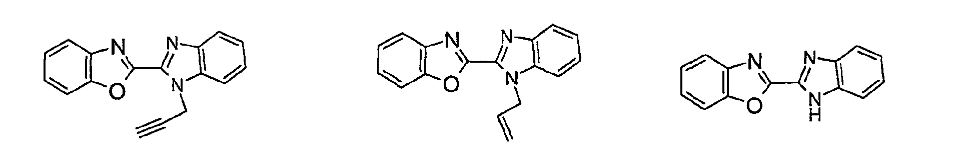 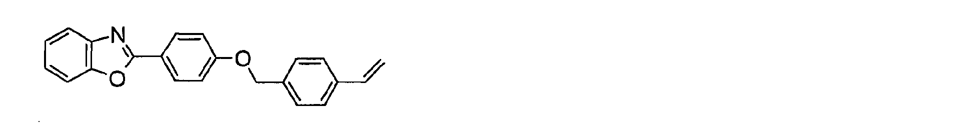 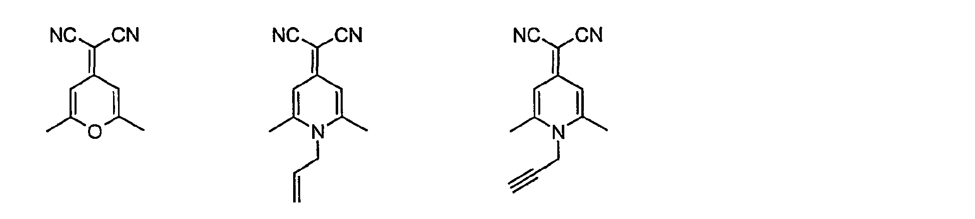 wherein R' is hydrogen, hydroxy, straight or branched chain C₁₋₂₀-alkyl, -alkoxy or C₂₋₂₀-alkenyl without being limited thereto.

It is also possible to prepare the crosslinkable chromophores by polymerizing one or more of the hydrolysable metal or semi-metal compounds, in particular the silane compounds mentioned above with each other or with other monomers such as the crosslinkable monomers with no UV filter activity defined below and then with a chromophore as defined above. In this case the crosslinkable chromophore is an oligomer which comprises one or more of the UV filter active moieties A. Usually such an oligomer is constituted from 50 monomer units or less, preferably from 20 monomer units or less, more preferably of 10 monomer units or less. Preferably such an oligomeric crosslinkable chromophore comprises 5 or less UV filter active moieties A, more preferably 3 or less, most preferably 1 UV filter active moiety A.

The crosslinkable chromophores with UV filter activity can be used for coating of the metal oxide particles per se or they can be used together with crosslinkable monomers which do not have UV filter activity. Preferably, the crosslinkable chromophores with UV filter activity are used together with crosslinkable monomers without UV filter activity, because this easily allows to adjust the amount of UV filter active moieties in the final coating. Alternatively, it is also possible to adjust the amount of UV filter active moieties in the final coating by using oligomers which have been prepared from monomers with UV filter activity and monomers with no UV filter activity as discussed above.

The crosslinkable monomers with no UV filter activity are preferably compounds of the formula M(R)ₙ(Q)_{q}, wherein M is a metallic or semi-metallic element such as silicon, titanium, zinc, aluminum or zirconium, preferably silicon, R is a hydrolysable substituent as defined above, preferably with not more than 10 carbon atoms, such as an alkoxide, an aryloxide, carboxylic ester, acyloxy group, diketonato group, hydrolysable aza group or a chlorine atom, preferably an alkoxide, an aryloxide or an acyloxy group, more preferably an alkoxide or an aryloxy group with 10 carbon atoms or less, in particular a C₁-C₆ alkoxide, most preferably methoxide or ethoxide, and n is an integer from 2 to 4, preferably 3 or 4. Q is as defined above, preferably octyl and q is 0 or 1, n+q=3 or 4, preferably 4 .

Particularly preferred are crosslinkable monomers such as tetraethoxysilane (TEOS), tetramethoxysilane (TEMOS), methyltriethoxysilane (METEOS), octyltrimethoxysilane (OTEOS) or partially hydrolyzed and/or partially condensed polymers (oligomers) thereof or a mixture of the above. Suitable crosslinkable monomers are also disclosed e.g. in EP-A 216 278.

It is possible that the coated metal oxide particles of the present invention contain two or more types of chromophores without the risk that there is an inactivation between the chromophore moieties, because the chromophore moieties are covalently bonded to the coating and therefore the risk of contact between the chromophore moieties is low. In a particular preferred embodiment of the present invention one or two kinds of crosslinkable chromophores are present in the coating, most preferably together with at least one kind of crosslinkable monomer which does not have any UV filter activity. The coated metal oxide particles of the present invention thus preferably contain one or two different kinds of UV filter active moieties A as defined above, in a preferred embodiment only one kind of chromophore moieties as defined above in a ratio of 1:50 to 10:1, more preferably of 1:5 to 1:30 to the crosslinkable monomer which does not have any UV filter activity. The ratio is based on a weight by weight basis.

The process for preparing the coated metal oxide particles of the present invention is not particularly restricted. All types of metal oxide particles which are principally known to be suitable for cosmetic or dermatological compositions can be used in the process, in particular particles of titanium dioxide, zinc oxide, zirconium oxide, iron oxide, cerium oxide and mixtures of these metal oxides. Furthermore, mixtures of these metal oxides with aluminum oxide and/or silicon dioxide can be used. The origin of the metal oxides is not particularly limited. Metal oxides can be prepared via a pyrogenic process, a sol-gel process, a grinding process, a plasma process, a precipitation process, a hydrothermal process or by a mining process or a combination of the above mentioned processes, to mention only the most common processes for preparing metal oxide particles. Titanium dioxide or Zinc oxide as well as mixtures of these metals oxides with aluminium oxide or with silicon dioxide are preferred.

The metal oxide particles which can be used in the coating process are either commercially available as e.g. pyrogenic titanium dioxide P25 from Degussa or can be prepared by methods known by a skilled person. Chemical mixtures of pyrogenic oxides can be prepared as described e.g. in EP-A 850 876. Examples of titanium silicon mixed oxides and titanium aluminium mixed oxides are disclosed in EP-A 609 533 and examples for silicon aluminium mixed oxides are disclosed e.g. in EP-A 1 084 617.

The crystalline form of the metal oxide may be of any crystal or amorphous type. For example, titanium dioxide may be any type of amorphous, rutil, anastase, brookite or a mixture thereof.

Particularly preferred are the metal oxides titanium dioxide or zinc oxide eventually doted with iron oxide.

The particle size of the particles to be coated according to the process of the present invention in not particularly limited. All particle sizes which are principally useful for incorporating into cosmetic or dermatological compositions can be coated with the coating composition according to the process of the present invention.

The coating of the metal oxide particles can be carried out by methods well known to a skilled person and described in the prior art and it can explicitly be referred to the methods disclosed in EP-A 988 853, in EP-A 1 284 277, in EP 581 651, in US 5 562 897 and in US 5 756 788. Preferred are sol-gel processes.

During the coating process the crosslinkable groups react to form a crosslinked polymer which forms the coating layer on the metal oxide particles.

An exemplary process for the coating of the metal oxide particles according to the present invention comprises the dispersion of the metal oxide particles in a suitable solvent, preferably in water with or without an emulsifier so that the dispersion comprises usually 1 to 80 wt.% of the metal oxide particles. To this dispersion a base or an inorganic acid is added under agitation, e.g. stirring and the crosslinkable chromophores and optionally the other crosslinkable monomers are added to the dispersion. Depending on the intended thickness of the coating the weight to weight ratio of crosslinkable monomers to metal oxide is usually between 0.005 and 1 more preferably between 0.01 and 0.3. The reaction product, the coated metal oxide particles, is separated and optionally washed and dried. The separation of the reaction product can be performed by usual processes such as filtration or centrifugation. The washing can be a washing with water or a suitable organic solvent or mixtures of water with organic solvent.

The coated metal oxide particles according to the invention can be dried using known methods such as those disclosed in Ullmann's Encyclopaedia of Industrial Chemistry. Other process steps can follow, such as calcinations, grinding procedures, granulation procedures or dispersion of the coated metal oxide particles in suitable liquids.

The temperature of the coating reaction is not critical. With water as a reaction medium the temperature is usually in the range of 10° to 80° C, preferably 40-80°C.

The quantity of base or acid required for the coating reaction is not critical and the best suitable amount of base or acid can be found by simple routine experiments. EP-A 1 284 277 mentions an amount of base in the range of 0.1 to 30 wt.% relative to the total reaction medium and in most cases this range is also suitable for the process of the present invention. A particularly suitable base or acid concentration is from 1 to 5 wt.%.

The base is not particularly restricted and the same bases can be used which are known from EP-A 1 284 277 such as ammonia, hydroxides such as sodium hydroxide, potassium hydroxide, or tetraalkylammonium hydroxide, carbonates such as ammonium carbonate, ammonium hydrogen carbonate, sodium carbonate or sodium hydrogen carbonate, organic bases such as amines, pyridines, anilines or guanidine, ammonium salts of carboxylic acids such as ammonium formate or ammonium acetate, alkyl ammonium salts of carboxylic acids such as monomethylamin formate or dimethylamin formate and mixtures thereof.

Particularly preferred are ammonia, ammonium carbonate, ammonium hydrogen carbonate, ammonium formate, ammonium acetate, sodium hydroxide, sodium carbonate and sodium hydrogen carbonate and mixtures of two or more of those bases.

In addition to bases or instead of bases inorganic acids such as for example hydrochloric acid, sulphuric acid or phosphoric acid and organic acids such as formic acid or acetic acid can be used or added if this facilitates the coating reaction.

In another process the metal oxides can be coated without using a solvent as e.g. described in US 5 756 788.

The primary particle size of the coated metal oxide particles is usually in the range from 2 to 100 nm, preferably in the range of 5 to 50 nm and the secondary particle size is preferably between 0.05 and 50 µm, preferably between 0.1 and 1 µm.

The coated metal oxide particles of the present invention can be included within cosmetic or dermatological compositions in manners well known to a skilled person. The cosmetic or dermatological compositions are generally topical cosmetic or dermatological compositions. For the preparation of the topical sunscreen compositions, especially preparations for dermatological and/or cosmetic use, such as skin protection and sunscreen formulations for everyday cosmetics the coated metal oxide particles of the present invention can be incorporated in auxiliary agents, e.g. a cosmetic base, which are conventionally used for such formulations. Where convenient, other conventional UV-A and/or UV-B and/ or broad spectrum screening agents may also be added. The combination of UV screens may show a synergistic effect. The amount of the coated metal oxide particles of the present invention and other known UV-screens is not critical. Suitable amounts of the coated metal oxide particles of the present invention are about 0.01 to about 50% by weight, preferably about 0.01 to 25 wt.% (depending on the payload and volume fraction of the particles) and about 0.5-12% by weight of at least one additional, hydrophilic and/or lipophilic UV-A or UV-B or broad spectrum screening agent. These additional screening agents are advantageously selected from among the compounds listed below without being limited thereto:

Examples of UV B or broad spectrum screening agents, i.e. substances having absorption maximums between about 290 and 340 nm, which come into consideration for combination with the coated particles of the present invention are for example the following organic and inorganic compounds:
- -- Acrylates such as 2-ethylhexyl 2-cyano-3,3-diphenylacrylate (octocrylene, PARSOL® 340), ethyl 2-cyano-3,3-diphenylacrylate and the like;
- -- Camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like;
- -- Cinnamate derivatives such as octyl methoxycinnamate (PARSOL® MCX), ethoxyethyl methoxycinnamate, diethanolamine methoxycinnamate (PARSOL® Hydro), isoamyl methoxycinnamate and the like as well as cinnamic acid derivatives bond to siloxanes;
- -- p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate,
- -- Benzophenones such as benzophenone-3, benzophenone-4, 2,2', 4, 4'-tetrahydroxy-benzophenone, 2,2'-dihydroxy-4,4'-dimethoxybenzophenone and the like;
- -- Esters of Benzalmalonic acid such as di-(2-ethylhexyl) 4-methoxybenzalmalonate;
- -- Esters of 2-(4-ethoxy-anilinomethylene)propandioic acid such as 2-(4-ethoxy anilinomethylene)propandioic acid diethyl ester as described in the European Patent Publication EP 0895 776;
- -- Organosiloxane compounds containing benzmalonate groups as described in the European Patent Publications EP 0358584 B1, EP 0538431 B1 and EP 0709080 A1;
- -- Drometrizole trisiloxane (Mexoryl XL);
- -- Pigments such as microparticulated TiO₂ and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.
- -- Imidazole derivatives such as e.g. 2-phenyl benzimidazole sulfonic acid and its salts (PARSOL®HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like.
- -- Salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like;
- -- Triazine derivatives such as octyl triazone (UVINUL T-150), dioctyl butamido triazone (UVASORB HEB), bis ethoxyphenol methoxyphenyl triazine (Tinosorb S) and the like;
- -- Encapsulated UV-filters such encapsulated as methoxycinnamate (Eusolex UV-pearls) and the like.

Examples of broad spectrum or UV A screening agents i.e. substances having absorption maximums between about 320 and 400 nm, which come into consideration for combination with the coated particles of the present invention are for example the following organic and inorganic compounds :
- -- Dibenzoylmethane derivatives such as 4-tert. butyl-4'-methoxydibenzoylmethane (PARSOL® 1789), dimethoxydibenzoylmethane, isopropyldibenzoylmethane and the like;
- -- Benzotriazole derivatives such as 2,2'-methylene-bis-(6-(2H-benzotriazole-2-yl)-4-(1,1,3,3,-tetramethylbutyl)-phenol (TINOSORB M) and the like;
- -- phenylene-1,4-bis-benzimidazolsulfonic acids or salts such as 2,2-(1,4-phenylene)bis-(1H-benzimidazol-4,6-disulfonic acid) (Neoheliopan AP);
- -- amino substituted hydroxybenzophenones such as 2-(4-Diethylamino-2-hydroxy-benzoyl)-benzoic acid hexylester as described in the European Patent Publication EP 1046391
- -- Pigments such as microparticulated ZnO or TiO₂ and the like. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

As dibenzoylmethane derivatives have limited photostability it may be desirable to photostabilize these UV-A screening agents. Thus, the term "conventional UV-A screening agent" also refers to dibenzoylmethane derivatives such as e.g. PARSOL® 1789 stabilized by, e.g.,
- -- 3,3-Diphenylacrylate derivatives as described in the European Patent Publications EP 0 514 491 B1 and EP 0 780 119 A1;
- -- Benzylidene camphor derivatives as described in the US Patent No. 5,605,680;
- -- Organosiloxanes containing benzmalonate groups as described in the European Patent Publications EP 0358584 B1, EP 0538431 B1 and EP 0709080 A1.

The compositions of the invention can also contain usual cosmetic adjuvants and additives, such as preservatives/ antioxidants, fatty substances/ oils, water, organic solvents, silicones, thickeners, softeners, emulsifiers, additional sunscreens, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, in particular those suited for providing an additional photoprotective effect by physically blocking out ultraviolet radiation, or any other ingredients usually formulated into cosmetics, in particular for the production of sunscreen/antisun compositions. The necessary amounts of the cosmetic and dermatological adjuvants and additives can, based on the desired product, easily be chosen by a skilled artisan in this field and will be illustrated in the examples, without being limited hereto.

An additional amount of antioxidants/ preservatives is generally preferred. Based on the invention all known antioxidants usually formulated into cosmetics can be used. Especially preferred are antioxidants chosen from the group consisting of amino acids (e.g. glycine, histidine, tyrosine, tryptophane) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-; oleyl-, y-linoleyl-, cholesteryl- and glycerylester) and the salts thereof, dilaurylthiodipropionate, distearylthiodipropionate, thiodipropionie acid and its derivatives (ester, ether, peptides, lipids, nucleotides, nucleosides and salts) as well as sulfoximine compounds (such as buthioninsulfoximine, homocysteinsulfoximine, buthioninsulfone, penta-, hexa-, heptathioninsulfoximine) in very low compatible doses (e.g. pmol bis µmol/kg), additionally (metal)-chelators (such as α-hydroxyfatty acids, palmic-, phytinic acid, lactoferrin), β-hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamin C and derivatives (such as ascorbylpalmitate and ascorbyltetraisopalmitate, Mg-ascorbylphosphate, Na- ascorbylphosphate, ascorbylacetate), tocopherole and derivates (such as vitamin-E-acetate), mixtures of nat. vitamin E, vitamin A and derivatives (vitamin-A-palmitate and -acetate) as well as coniferylbenzoate, rutinic acid and derivatives, α-glycosylrutin, ferulic acid, furfurylidenglucitol, carnosin, butylhydroxytoluene, butylhydroxyanisole, trihydroxybutyrophenone, urea and its derivatives, mannose and derivatives, zinc and derivatives (e.g. ZnO, ZnS0₄), Selen and derivatives (e.g. selenomethionin), stilbenes and derivatives (such as stilbenoxide, trans-stilbenoxide) and suitable derivatives (salts, esters, ethers, sugars, nucleotides, nucleosides, peptides and lipids) of the named active ingredients. One or more preservatives/antioxidants may be present in an amount about 0.01 wt.% to about 10 wt.% of the total weight of the composition of the present invention. Preferably, one or more preservatives/antioxidants are present in an amount about 0.1 wt.% to about 1 wt.%.

Typically formulations also contain surface active ingredients like emulsifiers, solubilizers and the like. An emulsifier enable two or more immiscible components to be combined homogeneously. Moreover, the emulsifier acts to stabilize the composition. Emulsifiers that may be used in the present invention in order to form O/W, W/O, O/W/O or W/O/W emulsions/ microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polygylceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and the salts thereof such as cetyl phosphate (Amphisol^{®} A), diethanolamine cetyl phosphate (Amphisol^{®}), potassium cetyl phosphate (Amphisol^{®} K), sodium glyceryl oleate phosphate, hydrogenated vegetable glycerides phosphate and mixtures thereof. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof. The preferred emulsifiers are cetyl phosphate (Amphisol^{®} A), diethanolamine cetyl phosphate (Amphisol^{®}), potassium cetyl phosphate (Amphisol^{®} K), PVP Eicosene copolymer, acrylates/C₁₀₋₃₀-alkyl acrylate crosspolymer, PEG-20 sorbitan isostearate, sorbitan isostearate, and mixtures thereof. The one or more emulsifiers are present in a total amount about 0.01 wt.% to about 20 wt.% of the total weight of the composition of the present invention. Preferably, about 0.1 wt.% to about 10 wt.% of emulsifiers are used.

The lipid phase can advantageously be chosen from:
mineral oils and mineral waxes;
oils such as triglycerides of caprinic acid or caprylic acid, preferable castor oil;
oils or waxes and other natural or synthetic oils, in an preferred embodiment esters of fatty acids with alcohols e.g. isopropanol, propyleneglycol, glycerine or esters of fatty alcohols with carbonic acids or fatty acids;
alkylbenzoates; and/or
silicone oils such as dimethylpolysiloxane, diethylpolysiloxane, diphenylpolysiloxane, cyclomethicones and mixtures thereof.

Exemplary fatty substances which can be incorporated in the oil phase of the emulsion, microemulsion, oleo gel, hydrodispersion or lipodispersion of the present invention are advantageously chosen from esters of saturated and/or unsaturated, linear or branched alkyl carboxylic acids with 3 to 30 carbon atoms, and saturated and/or unsaturated, linear and/or branched alcohols with 3 to 30 carbon atoms as well as esters of aromatic carboxylic acids and of saturated and/or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaureate, n-decyloleat, isooctylstearate, isononylstearate, isononylisononanoate, 2-ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for use in the formulation of the present invention include polar oils such as lecithins and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefins, hydrogenated polyisobutenes and isohexadecanes, favored polyolefins are polydecenes; dialkyl ethers such as dicaprylylether; linear or cyclic silicone oils such as preferably cyclomethicone (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in formulations of the present invention are isoeikosane; neopentylglycoldiheptanoate; propyleneglycoldicaprylate/ dicaprate; caprylic/ capric/ diglycerylsuccinate; butyleneglycol caprylat/caprat; C₁₂-₁₃-alkyllactate; di-C₁₂-₁₃ alkyltartrate; triisostearin; dipentaerythrityl hexacaprylat/hexacaprate; propyleneglycolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅-alkylbenzoate and isotridecylisononanoate as well as mixtures of C₁₂₋₁₅-alkylbenzoate, 2-ethylhexylisostearate and isotridecylisononanoate.

The oily phase of the formulation of the present invention can also contain natural vegetable or animal waxes such as bee wax, china wax, bumblebee wax and other waxes of insects as well as shea butter and cocoa butter.

A moisturizing agent may be incorporated into a composition of the present invention to maintain hydration or rehydrate the skin. Moisturizers that prevent water from evaporating from the skin by providing a protective coating are called emollients. Additionally an emollient provides a softening or soothing effect on the skin surface and is generally considered safe for topical use. Preferred emollients include mineral oils, lanolin, petrolatum, capric/caprylic triglyceraldehydes, cholesterol, silicones such as dimeticone, cyclometicone, almond oil, jojoba oil, avocado oil, castor oil, sesame oil, sunflower oil, coconut oil and grape seed oil, cocoa butter, olive oil aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅-alkyl benzoates, and mixtures thereof. The most preferred emollients are hydroxybenzoate esters, aloe vera, C₁₂₋₁₅-alkyl benzoates, and mixtures thereof. An emollient is present in an amount of about 1 wt.% to about 20 wt.% of the total weight of the composition. The preferred amount of emollient is about 2 wt.% to about 15 wt.%, and most preferably about 4 wt.% to about 10 wt.%.

Moisturizers that bind water, thereby retaining it on the skin surface are called humectants. Suitable humectants can be incorporated into a composition of the present invention such as glycerin, polypropylene glycol, polyethylene glycol, lactic acid, pyrrolidon carboxylic acid, urea, phospholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel®1000 (CAS-Nr. 178463-23-5) by SOLABIA S. One or more humectants are optionally present at about 0.5 wt.% to about 8 wt.% in a composition of the present invention, preferably about 1 wt.% to about 5 wt.%.

The aqueous phase of the compositions of the present invention can contain the usual cosmetic additives such as alcohols, especially lower alcohols, preferably ethanol and/ or isopropanol, low diols or polyols and their ethers, preferably propyleneglycol, glycerine, ethyleneglycol, ethyleneglycol monoethyl- or monobutylether, propyleneglycol monomethyl-or -monoethyl- or-monobutylether, diethyleneglycol monomethyl-or monoethylether and analogue products, polymers, foam stabilizers; electrolytes and especially one or more thickeners. Thickeners that may be used in formulations of the present invention to assist in making the consistency of a product suitable include carbomer, siliciumdioxide, magnesium and/ or aluminum silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole^{®} of type 980, 981, 1382, 2984, 5984 alone or mixtures thereof. Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 wt.% to about 8 wt.% in the composition of the present invention, preferably, 1 wt.% to about 5 wt.%.

The addition of electrolytes into the composition of the present invention may be necessary to change the behavior of a hydrophobic emulsifier. Thus, the emulsions/microemulsions of this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt.% to about 8 wt.% in the composition of the present invention.

The cosmetic compositions of the invention are useful as compositions for photoprotecting the human epidermis or hair against the damaging effect of ultraviolet irradiation, as sunscreen compositions. Such compositions can, in particular, be provided in the form of a lotion, a thickened lotion, a gel, a cream, a milk, an ointment, a powder or a solid tube stick and can be optionally be packaged as an aerosol and can be provided in the form of a mousse, foam or a spray. When the cosmetic composition according to the invention are provided for protecting the human epidermis against UV radiation or as sunscreen composition, they can be in the form of a suspension or dispersion in solvents or fatty substances, or alternatively in the form of an emulsion or microemulsion (in particular of O/W or W/O type, O/W/O or W/O/W-type), such as a cream or a milk, a vesicular dispersion, in the form of an ointment, a gel, a solid tube stick or an aerosol mousse. The emulsions can also contain anionic, nonionic, cationic or amphoteric surfactants.

The following examples are provided to further illustrate the processes and compositions of the present invention.

### Preparation of the crosslinkable chromophore

### Example 1

### 2-(4-Hydroxybenzylidene)malonic acid diethyl ester

A 250 ml round bottom flask, equipped with a reflux condenser, a Dean stark trap and an oil bath with a magnetic stirrer was charged with 10 g (81 mmol) 4-hydroxybenzaldehyde, 150 ml toluene, 0.97 ml (10 mmol) of piperidine and 0.8 g (6.5 mmol) benzoic acid are added, and the reaction is heated to 45°C. Then, 13.7 ml (90 mmol) diethyl malonate in 20 ml toluene is added drop wise. The reaction is refluxed and the water was collected in a Dean-Stark trap. After 16 hrs the reaction was cooled down, washed three times with water and once with diluted NaCl-solution. After drying over NaSO₄ the organic phase is concentrated and purified via column chromatography (hexane / EtOAc) yielding 18.6 g (86.2%) of 2-(4-hydroxybenzylidene)malonic acid diethyl ester.

### Example 2

2-[[4-[3-(triethoxysilyl)propyl)aminocarbonyl)oxy) benzylidene)malonic acid diethyl ester Into a stirred solution of 5 g (19 mmol) 2-(4-hydroxybenzylidene)-malonic acid diethyl ester prepared as described above in 70 ml of THF and a few drops of dibutyltindilaureate catalyst was added 5.6 ml (22 mmol) 3-isocyanatopropyl-triethoxysilane (ICTEOS) under nitrogen atmosphere. The reaction mixture was stirred at 40°C until no free 2-(4-hydroxybenzylidene)malonic acid diethyl ester could be detected by TLC. After evaporation of THF the crude product was purified via column chromatography (Hexane / EtOAc) yielding 8.7 g (90%) of 2-[[4-[3-(triethoxysilyl)propyl)aminocarbonyl)oxy) benzylidene)-malonic acid diethyl ester as colorless oil. UV (Ethanol), 290 nm (21068).

### Preparation of silica-coated titanium dioxide

The coating of the metal oxide with the crosslinkable chromophore with UV-A and/or UV-B and/or UV-C and/or broadband filter activity and optionally at least one type of crosslinkable monomer which does not have UV-A and/or UV-B and/or UV-C and/or broadband filter activity was verified by FT-IR spectrometry using a Perkin Elmer Spectrum One, FT-IR Spectrometer by analysis of the resulting transmission infrared absorption spectrum: a peak originating from the Si-O-Si stretching vibration was observed from 1000 to 1200 cm⁻¹. The incorporation of the crosslinkable chromophore is proven by a peak originating from the carbonyl group of the chromophore at 1650-1720 cm⁻¹. If octyl trimethoxysilane is used as crosslinkable monomer without UV filter activity an additional -CH₂/ -CH₃ stretching vibration is observed from 2800-3000 cm⁻¹.

The effectiveness of the coating is proven via the method described below showing a reduced coloration of all samples prepared according to the invention compared to uncoated titanium dioxide.

### Stability during irradiation

Untreated titanium dioxide produces an intense yellow coloration upon irradiation with UV-light. The more intense the color, the greater the reactivity of the titanium dioxide. This offers a good analytical test for the effectiveness of the surface treatment.

A 10% dispersion of the coated TiO₂ in Finsolv is drawn as a film on a glass plate with a 20 µm spreading knife. Afterwards irradiation was performed with a Hereaus Suntest with 40 MED. The judgement of the color of the samples was performed by comparison with "Methuen Handbook of Color", A. Kornerup und J.H. Wanscher, 3. edition, Eyre Methuen, London, 1984. Additionally, the samples were compared with untreated TiO₂ and with a commercially available silicone coated titanium dioxide grade: Uvinul^{®} TiO₂ (octylsilylated titanium dioxide from BASF).

| TiO₂ | Color* after irradiation with 40 MED |
|---|---|
| pyrogenic titanium dioxide (P25 ex Degussa) non treated | pastel yellow |
| Uvinul^{®} TiO₂ octylsilylated titanium dioxide | pale yellow |
| Example 3 | pale yellow |
| Example 4 | yellowish white |
| Example 5 | yellowish white |
| Example 6 | white |
| Example 7 | white |

| | |
|---|---|
| *according toTable 3 "Methuen Handbook of Color", A. Kornerup und J.H. Wanscher, 3. edition. | |

All samples showed a reduced coloration compared to the untreated TiO₂ and an equal or better performance compared to the commercial sample, Uvinul^{®} TiO₂ (octylsilylated titanium).

### Example 3

To 9g of pyrogenic titanium dioxide (P25, Degussa) 1g of a mixture of octyltrimethoxysilane and 2-[[4-[3-(triethoxysilyl)propyl)aminocarbonyl)oxy) benzylidene)malonic acid diethyl ester from example 2 in a ratio of 5:1 (w/w) is added. The powder is then mixed well and heated to 100°C. During this time ethanol and methanol are removed. When 97% of the theoretical amount (weight) of the alcohols are distilled off the reaction is cooled and the white solid is washed three times in consecutive order with N,N-dimethylformamide, methanol, dichloromethane and diethyl ether to remove unreacted silylation reagents. IR ν(C=O): 1649.

### Example 4

To 9g of pyrogenic titanium dioxide (P25, Degussa) 1g of a mixture of octyltrimethoxysilane and 2-[[4-[3-(triethoxysilyl)propyl)aminocarbonyl)oxy) benzylidene)malonic acid diethyl ester from example 2 in a ratio of 10:1 (w/w) is added. The powder is then mixed well and is heated to 100°C. During this time ethanol and methanol are removed. When 97% of the theoretical amount of the alcohols are distilled off the reaction is cooled down and the white solid is washed three times in consecutive order with N,N-dimethylformamide, methanol, dichloromethane and diethyl ether. IR ν(C=O): 1650.

### Example 5

To 9g of pyrogenic titanium dioxide (P25, Degussa) 1g of a mixture of octyltrimethoxysilane and 2-[[4-[3-(triethoxysilyl)propyl)aminocarbonyl)oxy) benzylidene)malonic acid diethyl ester from example 2 in a ratio of 15:1 (w/w) is added. The powder is then mixed well and is heated to 100°C. During this time ethanol and methanol are removed. When 97% of the theoretical amount of the alcohols are distilled a TLC is made (Hexane / Ethylacetate 1:1), which shows that all crosslinkable chromophore has disappeared. The reaction is cooled down and the white solid is washed three times in consecutive order with N,N-dimethylformamide, methanol, dichloromethane and diethyl ether. After drying the effectiveness of the coating is proven via irradiation at 40 MED showing a reduced coloration compared to uncoated titanium dioxide. IR ν(C=O)_{:} 1651.

### Example 6

To a dispersion of 5g pyrogenic titanium dioxide (P25, Degussa) in 70 ml water, 5g of a mixture of tetraethoxysilane (TEOS) and 2-[[4-[3-(triethoxysilyl)propyl)aminocarbonyl)oxy)-benzylidene)malonic acid diethyl ester from example 2 in a ratio of 5:1 (w/w) is added and stirred at room temperature for 15 minutes. Afterwards, 1.5 ml of aqueous ammonium hydroxide solution (28-30 wt%) is added and the reaction mixture is stirred at 80°C overnight. The suspension is filtered and the slightly grey solid is washed three times in consecutive order with N,N-dimethylformamide, methanol, dichloromethane and diethyl ether. IR ν(C=O): 1650.

### Example 7

To a dispersion of 5g of pyrogenic titanium dioxide (P25, Degussa) in 50g of an aqueous solution of 1% of cetyltriammonium chloride in water, 2g of a mixture of tetraethoxysilane (TEOS) and 2-[[4-[3-(triethoxysilyl)propyl)aminocarbonyl)oxy)benzylidene)malonic acid diethyl ester from example 2 in a ratio of 5:1 (w/w) is added and stirred at room temperature for 15 minutes. Afterwards 50g of an aqueous solution of sodium hydroxide at pH 11.3 is added and the resulting mixture is stirred at room temperature for 1 hour. The suspension is filtered and the slightly yellow solid is washed three times in consecutive order with N,N-dimethylformamide, methanol, dichloromethane and diethyl ether. IR ν(C=O): 1710.

### Example 8

### O/W sun milk

| | Ingredients | INCI Nomenclature | % w/w |
|---|---|---|---|
| A) | PARSOL SLX | Dimethico Diethylbenzalmalonate Polysilicone-15 | 6.00 |
| | Neo Heliopan AP | | 3.00 |
| | Tinosorb S | Hydrogenated Cocoglycerides | 3.00 |
| | Lanette O | Cetearyl Alcohol | 2.00 |
| | Myritol 318 | Caprylic/capric Triglyceride | 6.00 |
| | Mineral oil | Mineral oil | 2.00 |
| | Vitamin E acetate | Tocopheryl Acetate | 1.00 |
| | Prisorine 3515 | Isostearyl Alcohol | 4.00 |
| B) | Edeta BD | Disodium EDTA | 0.10 |
| | Phenonip | Phenoxyethanol & Methylparaben & Ethylparaben & Propylparaben & Butylparaben | 0.60 |
| | Amphisol K | Potassium Cetyl Phosphate | 2.00 |
| | Water deionized | Aqua | ad100 |
| | 1,2-Propylene Glycol | Propylene Glycol | 5.00 |
| | Carbopol 981 | Carbomer | 0.30 |
| | Tinosorb M | Methylene Bis-Benzotriazolyl Tetramethylbutylphenol | 6.00 |
| | KOH 10% solution | Potassium Hydroxyde | 2.10 |
| C) | Chromophore coated metal oxide | | 1-20 |

### Procedure:

Heat part A) and B) to 85°C while stirring. When homogeneous, add part B) to A) under agitation. Cool to ambient temperature while stirring and add part C). Homogenize to achieve a small particle size.

### Example 9

Sun milk waterproofed

| | Ingredients | INCI Nomenclature | % w / w |
|---|---|---|---|
| A) | PARSOL SLX | Polysilicone-15Dimethico Diethylbenzalmalonate | 6.00 |
| | PARSOL 1789 | Butyl Methoxydibenzoylmethane | 2.00 |
| | PARSOL 5000 | 4-Methylbenzylidene Camphor | 4.00 |
| | Uvinul T 150 | Ethylhexyltriazone | 2.00 |
| | Silicone DC 200/350 cs | Dimethicone | 1.00 |
| | Lanette O | Cetearyl Alcohol | 2.00 |
| | Softisan 100 | Hydrogenated Coco-Glycerides | 3.00 |
| | Tegosoft TN | C12-15 Alkyl Benzoate | 6.00 |
| | Cetiol B | Dibutyl Adipate | 7.00 |
| | Vitamin E acetate | Tocopheryl Acetate | 2.00 |
| | BHT | BHT | 0.05 |
| | Edeta BD | Disodium EDTA | 0.10 |
| | Phenonip | Phenoxyethanol & Methylparaben & Ethylparaben & Propylparaben & Butylparaben | 0.60 |
| | Amphisol | Cetyl Phosphate DEA | 2.00 |
| B) | Water deionized | Aqua | ad 100 |
| | Propylene Glycol | Propylene Glycol | 5.00 |
| | Carbopol 980 | Carbomer | 0.30 |
| | KOH (10% sol.) | Potassium Hydroxide | 1.50 |
| C) | Chromophore coated metal oxide | | 1-20 |

### Procedure:

Heat part A) and B) to 85°C while stirring. When homogeneous, add part B) to A) under agitation. Cool to ambient temperature while stirring and add part C). Homogenize to achieve a small particle size.

### Example 10

### Sun milk for babies and children

| | Ingredients | INCI Nomenclature | % w / w |
|---|---|---|---|
| A) | Tegosoft TN | C12-15 Alkyl Benzoate | 5.00 |
| | Silicone 2503 Cosmetic Wax | Stearyl Dimethicone | 2.00 |
| | Cetyl Alcohol | Cetyl Alcohol | 1.00 |
| | Butylated Hydroxytoluene | BHT | 0.05 |
| | Estol GMM 3650 | Glyceryl Myristate | 4.00 |
| | Edeta BD | Disodium EDTA | 0.10 |
| | Phenonip | Phenoxyethanol & Methylparaben & Ethylparaben & Propylparaben & Butylparaben | 0.60 |
| | Amphisol A | Cetyl Phosphate | 2.00 |
| B) | Water deionized | Aqua | ad 100 |
| | Carbopol 980 | Carbomer | 0.6 |
| | Glycerine | Glycerine | 3.00 |
| | KOH sol. 10% | Potassium Hydroxide | 2.4 |
| C) | Chromophore coated metal oxide | | 1-20 |

### Procedure:

Heat part A) and B) to 85°C while stirring. When homogeneous, add part B) to A) under agitation. Cool to ambient temperature while stirring and add part C). Homogenize to achieve a small particle size.

### Example 11

### High protective sun milk

| | Ingredients | INCI Nomenclature | % w / w |
|---|---|---|---|
| A) | PARSOL SLX | Polysilicone-15Dimethico Diethylbenzalmalonate | 6.00 |
| | PARSOL 1789 | Butyl Methoxydibenzoylmethane | 2.00 |
| | PARSOL 5000 | 4-Methylbenzylidene Camphor | 4.00 |
| | Uvinul T 150 | | 2.00 |
| | Silicone DC 200/350 cs | Dimethicone | 1.00 |
| | Lanette O | Cetearyl Alcohol | 2.00 |
| | Softisan 100 | Hydrogenated Coco-Glycerides | 3.00 |
| | Tegosoft TN | C12-15 Alkyl Benzoate | 6.00 |
| | Cetiol B | Dibutyl Adipate | 7.00 |
| | Vitamin E acetate | Tocopheryl Acetate | 2.00 |
| | BHT | BHT | 0.05 |
| | Edeta BD | Disodium EDTA | 0.10 |
| | Phenonip | Phenoxyethanol & Methylparaben & Ethylparaben & Propylparaben & Butylparaben | 0.60 |
| | Amphisol K | Potassium Cetyl Phosphate | 2.00 |
| B) | Water deionized | Aqua | ad 100 |
| | Propylene Glycol | Propylene Glycol | 5.00 |
| | Carbopol 980 | Carbomer | 0.30 |
| | KOH (10% sol.) | Potassium Hydroxide | 1.50 |
| C) | Chromophore coated metal oxide | | 1-20 |
| D) | Perfume | Perfume | q.s. |

### Procedure:

Heat part A) and B) to 85°C while stirring. When homogeneous, add part B) to A) under agitation. Cool to ambient temperature while stirring and add part C) and D). Homogenize to achieve a small particle size.

### Example 12

### Water-free sun gel

| | Ingredients | INCI Nomenclature | % w / w |
|---|---|---|---|
| A) | PARSOL MCX | Ethylhexyl Methoxycinnamate | 6.00 |
| | PARSOL 1789 | Butyl Methoxydibenzoylmethane | 4.00 |
| | PARSOL 5000 | 4-Methylbenzylidene Camphor | 4.00 |
| | Uvasorb HEB | Diethylhexyl Butamido Triazone | 1.50 |
| | Uvinul A plus | | 2.00 |
| | Vitamin E acetate | Tocopheryl Acetate | 1.50 |
| | Tegosoft TN | C12-15 Alkyl Benzoate | 9.00 |
| | Elefac I-205 | Ethylhexyldodecyl Neopentanoate | 2.00 |
| | Alcohol | Alcohol | ad 100 |
| | Isopropyl Alcohol | Isopropyl Alcohol | 20.00 |
| B) | Klucel MF | Hydroxypropylcellulose | 2.00 |
| C) | Chromophore coated metal oxide | | 1-20 |
| D) | perfume | | q.s. |

### Procedure:

Mix part A) and B) while stirring. When homogeneous, add part C) and D) under agitation.

### Example 13

### Sun gel

| | Ingredients | INCI Nomenclature | % w / w |
|---|---|---|---|
| A) | Pemulen TR-2 | Acrylates/C10-30 Alky Acrylate Crosspolymer | 0.60 |
| | Phenonip | Phenoxyethanol & Methylparaben & Ethylparaben & Propylparaben & Butylparaben | 0.60 |
| | Edeta BD | Disodium EDTA | 0.1 |
| | Aqua | Aqua | ad 100 |
| B) | PARSOL 1789 | Butyl Methoxydibenzoylmethane | 4.00 |
| | PARSOL 340 | Octocrylene | 3.00 |
| | Tegosoft TN | C12-15 Alkyl Benzoate | 15.00 |
| | Antaron V-216 | PVP/Hexadecene Copolymer | 1.00 |
| | Vitamin E acetate | Tocopheryl Acetate | 0.50 |
| | Butylated Hydroxytoluene | BHT | 0.05 |
| | Cremophor RH 410 | PEG-40 Hydrogenated Castor Oil | 0.50 |
| | Tris Amino | Tromethamine | 0.50 |
| C) | Chromophore coated metal oxide | | 1-20 |
| D) | Perfume | Perfume | q.s. |

### Procedure:

Heat part A) and B) to 85°C while stirring. When homogeneous, add part B) to A) under agitation. Cool to ambient temperature while stirring and add part C) and D). Homogenize to achieve a small particle size.

### Example 14

### High protection WO sun milk

| | Ingredients | INCI Nomenclature | % w / w |
|---|---|---|---|
| A) | PARSOL 1789 | Butyl Methoxydibenzoylmethane | 2.00 |
| | PARSOL 5000 | 4-Methylbenzylidene Camphor | 4.00 |
| | Uvinul T 150 | Ethylhexyl Triazone | 2.00 |
| | Uvinul TiO2 | Titanium Dioxide and Trimethoxycaprylylsilane | 5.00 |
| | Arlacel P 135 | PEG-30 Dipolyhydroxystearate | 2.00 |
| | Tegosoft TN | C12-15 Alkyl Benzoate | 5.00 |
| | Cosmacol EMI | Di-C12-13 Alkyl Malate | 6.00 |
| | Miglyol 840 | Propylene Glycol Dicaprylate/Dicaprate | 6.00 |
| | Butylated Hydroxytoluene | BHT | 0.05 |
| | Phenonip | Phenoxyethanol & Methylparaben & Ethyl-paraben & Propylparaben & Butylparaben | 0.60 |
| B) | Deionized water | Aqua | ad 100 |
| | Glycerin | Glycerin | 5.00 |
| | Edeta | Disodium EDTA | 0.1 |
| | NaCl | Sodium Chloride | 0.30 |
| C) | PARSOL HS | Phenylbenzyimidazole Sulphonic Acid | 4.00 |
| | Water | Aqua | 20.00 |
| | Triethanolamine 99%. | Triethanolamine | 2.50 |
| D) | Chromophore coated metal oxide | | 1-20 |
| E) | Perfume | | q.s. |

### Procedure:

Heat part A), B) and C) to 85°C while stirring. When homogeneous, add part B) and C) to A) under agitation. Cool to ambient temperature while stirring and add part D) and E). Homogenize to achieve a small particle size.

### Example 15

### W/O milk with pigments

| | Ingredients | INCI Nomenclature | % w / w |
|---|---|---|---|
| A) | Cremophor WO 7 | PEG-7 Hydrogenated Castor Oil | 6.00 |
| | Elfacos ST 9 | PEG-45/Dodecyl Glycol Copolymer | 2.00 |
| | PARSOL 1789 | Butyl Methoxydibenzoylmethane | 3.00 |
| | Tinosorb S | | 5.00 |
| | PARSOL 5000 | 4-Methylbenzylidene Camphor | 4.00 |
| | microfine ZnO | Zinc Oxide | 2.00 |
| | Microcrystalline wax | Microcrystalline Wax | 2.00 |
| | Miglyol 812 | Caprylic/capric Triglyceride | 5.00 |
| | Vitamin E acetate | Tocopheryl Acetate | 1.00 |
| | Jojoba oil | Simmondsia Chinensis Seed Oil | 5.00 |
| | Edeta BD | Disodium EDTA | 0.10 |
| | Butylated Hydroxytoluene | BHT | 0.05 |
| | Phenonip | Phenoxyethanol & Methylparaben & Ethyl-paraben & Propylparaben & Butylparaben | 0.60 |
| B) | Water deionized | Aqua | ad 100 |
| | Glycerin | Glycerin | 5.00 |
| C) | Neo Heliopan AP | | 2.00 |
| | Water deionized | Aqua | 20.00 |
| | KOH 10% solution | Potassium Hydroxide | 4.00 |
| D) | Chromophore coated metal oxide | | 1-20 |
| E) | Perfume | Perfume | q.s. |

### Procedure:

Heat part A), B) and C) to 85°C while stirring. When homogeneous, add part B) and C) to A) under agitation. Cool to ambient temperature while stirring and add part D) and E). Homogenize to achieve a small particle size.

### Example 16

### Protective Day cream with Vitamin C

| | Ingredients | INCI Nomenclature | % w / w |
|---|---|---|---|
| A) | PARSOL SLX | Polysilicone-15Dimethico Diethylbenzalmalonate | 4.00 |
| | PARSOL 1789 | Butyl Methoxydibenzoylmethane | 1.50 |
| | Glyceryl Myristate | Glyceryl Myristate | 2.00 |
| | Cetyl Alcohol | Cetyl Alcohol | 0.50 |
| | Myritol 318 | Caprylic/Capric Triglyceride | 5.00 |
| | Crodamol DA | Diisopropyl Adipate | 5.00 |
| | Vitamin E acetate | Tocopheryl Acetate | 2.00 |
| | Butylated Hydroxytoluene | BHT | 0.05 |
| | Phenonip | Phenoxyethanol & Methylparaben & Ethylparaben & Propylparaben & Butylparaben | 0.60 |
| | Edeta BD | Disodium EDTA | 0.10 |
| | Amphisol K | Potassium Cetyl Phosphate | 2.00 |
| B) | Water deionized | Aqua | ad 100 |
| | 1,2-Propylene Glycol | Propylene Glycol | 2.00 |
| | D-Panthenol 75 L | Panthenol | 2.00 |
| | Ethanol | Ethanol | 5.00 |
| | Allantoin | Allantoin | 0.20 |
| | Carbopol ETD 2001 | Carbomer | 0.30 |
| | KOH 10% sol. | Potassium Hydroxide | 1.50 |
| C) | Water | Aqua | 10.00 |
| | Stay-C 50 | Sodium Ascorbyl Phosphate | 0.50 |
| D) | Chromophore coated metal oxide | | 1-20 |
| E) | Perfume | Perfume | q.s. |

## Claims

1. Process for producing coated metal oxide particles, wherein metal oxide particles are coated with at least one type of crosslinkable chromophore with UV-A and/or UV-B and/or UV-C and/or broadband filter activity which is a monomer of the formula M(R)ₙ(P)ₘ(Q)_{q}, wherein M is selected from the group consisting of silicon, titanium, zinc, aluminium and zirkonium, R is a hydrolysable group, P is a chromophore with UV-A, UV-B and/or UV-C and/or broadband filter activity, Q is a non-hydrolysable group, n is 2 or 3, m is 1 or 2 and q is 0 or 1, wherein n+m+q=4 and optionally at least one type of crosslinkable monomer which does not have UV-A and/or UV-B and/or UV-C and/or broadband filter activity.

2. Process for producing coated metal oxide particles according to claim 1, wherein metal oxide particles are coated with at least one type of crosslinkable chromophores with UV-A and/or UV-B and/or UV-C and/or broadband filter activity and at least one type of crosslinkable monomers which does not have UV-A and/or UV-B and/or UV-C and/or broadband filter activity.

3. Process for producing coated metal oxide particles according to claim 1 or claim 2, wherein the metal oxide particles are coated by a sol-gel process.

4. Process for producing coated metal oxide particles according to any of claims 1 to 3, wherein the metal oxide particles are coated by contacting them in the form of an aqueous dispersion in the presence of a base with at least one type of crosslinkable chromophores with UV-A and/or UV-B and/or UV-C and/or broadband filter activity and optionally at least one type of crosslinkable monomers which does not have UV-A and/or UV-B and/or UV-C and/or broadband filter activity

5. Process for producing coated metal oxide particles according to claim 1, wherein the chromophore P has the general formula A-(B)_{b}(C)_{c}(D)_{d}(E)ₑ- which is chemically bonded to M
wherein
**A** is a chromophore with UV-A and/or UV-B filter and/or broadband activity and - **(B)_{b}(C)_{c}(D)_{d}(E)ₑ-** is a spacer group in which
**B** is a linear or branched alkylene group with up to 20 carbon atoms
**C** is 0, S or NH
**D** is a CONH- group
**E** is a linear or branched alkylene or alkenylene group with up to 20 carbon atoms and
**b** is 0 or 1,
**c** is 0 or 1,
**d** is 0 or 1 and
**e** is 0 or 1.

6. Process for producing coated metal oxide particles according to claim 5, wherein M is silicon.

7. Process for producing coated metal oxide particles according to claim 6, wherein all crosslinkable compounds used for producing the coated metal oxide particles are silicon-containing monomers.

8. Process for producing coated metal oxide particles according to any of claims 1 to 6, wherein at least one type of crosslinkable chromophore with UV-A and/or UV-B and/or UV-C and/ or broadband filter activity is a silane monomer comprising at least two C₁₋₆-alkoxy groups.

9. Process for producing coated metal oxide particles according to claim 8, wherein all monomers which are used for producing metal oxide particles are silane monomers comprising at least two C₁₋₆-alkoxy groups.

10. Process for producing coated metal oxide particles according to any of claims 1 to 9, wherein the amount of crosslinkable chromophores with UV-A and/or UV-B and/or UV-C and/ or broadband filter activity is such that the concentration of UV absorber moieties in the final coating is 0.05-20 % by weight.

11. Process for producing coated metal oxide particles according to any of claims 1 to 10, wherein the metal oxide is selected from the group of titanium dioxide, zinc oxide, zircon oxide, iron oxide, cerium oxide, mixtures of the above and mixtures of the above with aluminum oxide, and/or silicium dioxide.

12. Process for producing coated metal oxide particles according to claim 11, wherein the metal oxide is titanium dioxide or zinc oxide or a mixture of the above or a mixture of the above with aluminum oxide, and/or iron oxide, and/or silicium dioxide.

13. Process for producing coated metal oxide particles according to any of claims 1 to 12, wherein the primary particle size of the coated oxide particles is in the range from 2 to 100 nm.

14. Coated metal oxide particles obtainable according to the process of any of claims 1 to 13.

15. Cosmetic or dermatological composition comprising the coated metal oxide particles as defined in claim 14.

16. Cosmetic or dermatological composition according to claim 15 wherein additionally further UV-A screening agent/s and/or UV-B screening agent/s and/ or broadband screening agent/s are present.

17. Cosmetic or dermatological composition according to claims 15 or 16 comprising from 0.01 % - 25 % by weight of the coated metal oxide particles.

18. Use of the coated metal oxide particles as defined in claim 14 for the stabilization of UV-filters

19. Use according to claim 18, wherein the UV-filter is dibenzoyl methane or a derivative thereof.

20. Use of the coated metal oxide particles according to claim 14 for the protection of human skin or hair against UV-radiation.

## Patentansprüche

1. Verfahren zur Herstellung beschichteter Metalloxidteilchen, bei dem Metalloxidteilchen mit mindestens einer Art vemetzbarem Chromophor mit UV-A- und/oder UV-B- und/oder UV-C- und/oder Breitbandfilteraktivität, der ein Monomer der Formel M(R)ₙ(P)ₘ(Q)_{q} ist, wobei M aus der Gruppe ausgewählt ist, bestehend aus Silicium, Titanium, Zink, Aluminium und Zirkonium, R eine hydrolysierbare Gruppe ist, P ein Chromophor mit UV-A-, UV-Bund/oder UV-C- und/oder Breitbandfilteraktivität ist, Q eine nicht hydrolysierbare Gruppe ist, n 2 oder 3 ist, m 1 oder 2 ist und q 0 oder 1 ist, wobei n + m + q = 4, und gegebenenfalls mindestens einer Art vemetzbarem Monomer ohne UV-A- und/oder UV-B- und/oder UV-C-und/oder Breitbandfilteraktivität beschichtet werden.

2. Verfahren zur Herstellung beschichteter Metalloxidteilchen nach Anspruch 1, bei dem Metalloxidteilchen mit mindestens einer Art vemetzbarem Chromophor mit UV-A- und/oder UV-B- und/oder UV-C- und/oder Breitbandfilteraktivität und mindestens einer Art vemetzbarem Monomer ohne UV-A- und/oder UV-B- und/oder UV-C- und/oder Breitbandfilteraktivität beschichtet werden.

3. Verfahren zur Herstellung beschichteter Metalloxidteilchen nach Anspruch 1 oder Anspruch 2, wobei die Metalloxidteilchen durch ein Sol-Gel-Verfahren beschichtet werden.

4. Verfahren zur Herstellung beschichteter Metalloxidteilchen nach einem der Ansprüche 1 bis 3, wobei die Metalloxidteilchen durch Kontaktieren dieser in Form einer wässerigen Dispersion in Gegenwart einer Base mit mindestens einer Art vernetzbarem Chromophor mit UV-A- und/oder UV-B- und/oder UV-C- und/oder Breitbandfilteraktivität und gegebenenfalls mindestens einer Art vernetzbarem Monomer ohne UV-A- und/oder UV-B- und/oder UV-C- und/oder Breitbandfilteraktivität beschichtet werden.

5. Verfahren zur Herstellung beschichteter Metalloxidteilchen nach Anspruch 1, wobei der Chromophor P, der chemisch an M gebunden ist, die allgemeine Formel A-(B)_{b}(C)_{c}(D)_{d}(E)ₑ-aufweist, wobei
A ein Chromophor mit UV-A- und/oder UV-B-Filter- und/oder Breitbandaktivität ist und -(B)_{b}(C)_{c}(D)_{d}(E)ₑ- eine Spacergruppe ist, in der
B eine lineare oder verzweigte Alkylengruppe mit bis zu 20 Kohlenstoffatomen ist,
C O, S oder NH ist,
D eine CONH-Gruppe ist,
E eine lineare oder verzweigte Alkylen- oder Alkenylengruppe mit bis zu 20 Kohlenstoffatomen ist und
b 0 oder 1 ist,
c 0 oder 1 ist,
d 0 oder 1 ist und
e 0 oder 1 ist.

6. Verfahren zur Herstellung beschichteter Metalloxidteilchen nach Anspruch 5, wobei M Silicium ist.

7. Verfahren zur Herstellung beschichteter Metalloxidteilchen nach Anspruch 6, wobei alle vernetzbaren Verbindungen, die zur Herstellung der beschichteten Metalloxidteilchen verwendet werden, Silicium enthaltende Monomere sind.

8. Verfahren zur Herstellung beschichteter Metalloxidteilchen nach einem der Ansprüche 1 bis 6, wobei mindestens eine Art von vemetzbarem Chromophor mit UV-A- und/oder UV-Bund/oder UV-C- und/oder Breitbandfilteraktivität ein Silanmonomer, umfassend mindestens zwei C₁₋₆-Alkoxygruppen, ist.

9. Verfahren zur Herstellung beschichteter Metalloxidteilchen nach Anspruch 8, wobei alle Monomere, die zur Herstellung von Metalloxidteilchen verwendet werden, Silanmonomere, umfassend mindestens zwei C₁₋₆-Alkoxygruppen, sind.

10. Verfahren zur Herstellung beschichteter Metalloxidteilchen nach einem der Ansprüche 1 bis 9, wobei die Menge an vernetzbaren Chromophoren mit UV-A- und/oder UV-B- und/oder UV-C- und/oder Breitbandfilteraktivität derart ist, dass die Konzentration an UV-Absorberkomponenten in der fertigen Beschichtung 0,05 - 20 Gew.-% beträgt.

11. Verfahren zur Herstellung beschichteter Metalloxidteilchen nach einem der Ansprüche 1 bis 10, wobei das Metalloxid aus der Gruppe von Titandioxid, Zinkoxid, Zirkoniumoxid, Eisenoxid, Ceroxid, Gemischen der obigen und Gemischen der obigen mit Aluminiumoxid und/oder Siliciumdioxid ausgewählt ist.

12. Verfahren zur Herstellung beschichteter Metalloxidteilchen nach Anspruch 11, wobei das Metalloxid Titandioxid oder Zinkoxid oder ein Gemisch der obigen oder ein Gemisch der obigen mit Aluminiumoxid und/oder Eisenoxid und/oder Siliciumdioxid ist.

13. Verfahren zur Herstellung beschichteter Metalloxidteilchen nach einem der Ansprüche 1 bis 12, wobei die primäre Teilchengröße der beschichteten Oxidteilchen im Bereich von 2 bis 100 nm liegt.

14. Beschichtete Metalloxidteilchen, erhältlich durch ein Verfahren nach einem der Ansprüche 1 bis 13.

15. Kosmetische oder dermatologische Zusammensetzung, umfassend die beschichteten Metalloxidteilchen, wie in Anspruch 14 definiert.

16. Kosmetische oder dermatologische Zusammensetzung nach Anspruch 15, wobei außerdem weitere UV-A-Schutzmittel und/oder UV-B-Schutzmittel und/oder Breitband-Schutzmittel vorliegen.

17. Kosmetische oder dermatologische Zusammensetzung nach den Ansprüchen 15 und 16, umfassend 0,01 Gew.-% - 25 Gew.-% der beschichteten Metalloxidteilchen.

18. Verwendung der beschichteten Metalloxidteilchen, wie in Anspruch 14 definiert, für die Stabilisierung von UV-Filtern.

19. Verwendung nach Anspruch 18, wobei der UV-Filter Dibenzoylmethan oder ein Derivat davon ist.

20. Verwendung der beschichteten Metalloxidteilchen nach Anspruch 14 zum Schutz von menschlicher Haut oder menschlichem Haar gegen UV-Strahlung.

## Revendications

1. Procédé de production de particules d'oxyde métallique revêtues, dans lequel les particules d'oxyde métallique sont revêtues d'au moins un type de chromophore réticulable ayant une activité de filtre UV-A et/ou UV-B et/ou UV-C et/ou à large bande, qui est un monomère de formule M(R)ₙ(P)ₘ(Q)_{q} dans laquelle M est choisi dans le groupe constitué du silicium, du titane, du zinc, de l'aluminium et du zirconium, R est un groupement hydrolysable, P est un chromophore ayant une activité de filtre UV-A, UV-B et/ou UV-C et/ou à large bande, Q est un groupement non hydrolysable, n vaut 2 ou 3, m vaut 1 ou 2 et q vaut 0 ou 1, dans laquelle n + m + q = 4 et, éventuellement, d'au moins un type de monomère réticulable qui n'exerce pas d'activité de filtre UV-A et/ou UV-B et/ou UV-C et/ou à large bande.

2. Procédé de production de particules d'oxyde métallique revêtues selon la revendication 1, dans lequel des particules d'oxyde métallique sont revêtues d'au moins un type de chromophores réticulables ayant une activité de filtre UV-A et/ou UV-B et/ou UV-C et/ou à large bande et d'au moins un type de monomères réticulables qui n'exerce pas d'activité de filtre UV-A et/ou UV-B et/ou UV-C et/ou à large bande.

3. Procédé de production de particules d'oxyde métallique revêtues selon la revendication 1 ou la revendication 2, dans lequel les particules d'oxyde métallique sont revêtues par un procédé sol-gel.

4. Procédé de production de particules d'oxyde métallique revêtues selon l'une quelconque des revendications 1 à 3, dans lequel les particules d'oxyde métallique sont revêtues en mettant celles-ci en contact, sous la forme d'une dispersion aqueuse en présence d'une base, avec au moins un type de chromophores réticulables ayant une activité de filtre UV-A et/ou UV-B et/ou UV-C et/ou à large bande et, éventuellement, au moins un type de monomères réticulables qui n'exerce pas d'activité de filtre UV-A et/ou UV-B et/ou UV-C et/ou à large bande.

5. Procédé de production de particules d'oxyde métallique revêtues selon la revendication 1, dans lequel le chromophore P répond à la formule générale A-(B)_{b}(C)_{c}(D)_{d}(E)ₑ- qui est chimiquement liée à M
dans laquelle
A est un chromophore ayant une activité de filtre UV-A et/ou UV-B et/ou à large bande et la partie -(B)_{b}(C)_{c}(D)_{d}(E)ₑ- représente un groupement d'espacement dans lequel
B est un groupement alkylène linéaire ou ramifié ayant jusqu'à 20 atomes de carbone
C représente O, S ou NH
D est un groupement CONH-
E est un groupement alkylène ou alcénylène linéaire ou ramifié ayant jusqu'à 20 atomes de carbone
et
b vaut 0 ou 1,
c vaut 0 ou 1,
d vaut 0 ou 1 et
e vaut 0 ou 1.

6. Procédé de production de particules d'oxyde métallique revêtues selon la revendication 5, dans lequel M est le silicium.

7. Procédé de production de particules d'oxyde métallique revêtues selon la revendication 6, dans lequel tous les composés réticulables utilisés pour la production des particules d'oxyde métallique revêtues, sont des monomères contenant du silicium.

8. Procédé de production de particules d'oxyde métallique revêtues selon l'une quelconque des revendications 1 à 6, dans lequel au moins un type de chromophore réticulable ayant une activité de filtre UV-A et/ou UV-B et/ou UV-C et/ou à large bande est un monomère de silane comprenant au moins deux groupements alcoxy en C₁₋₆.

9. Procédé de production de particules d'oxyde métallique revêtues selon la revendication 8, dans lequel tous les monomères qui sont utilisés pour la production des particules d'oxyde métallique sont des monomères de silane comprenant au moins deux groupements alcoxy en C₁₋₆.

10. Procédé de production de particules d'oxyde métallique revêtues selon l'une quelconque des revendications 1 à 9, dans lequel la quantité de chromophores réticulables ayant une activité de filtre UV-A et/ou UV-B et/ou UV-C et/ou à large bande est telle que la concentration des résidus absorbant les UV dans le revêtement final est de 0,05 à 20 % en poids.

11. Procédé de production de particules d'oxyde métallique revêtues selon l'une quelconque des revendications 1 à 10, dans lequel l'oxyde métallique est choisi dans le groupe constitué du dioxyde de titane, de l'oxyde de zinc, de l'oxyde de zirconium, de l'oxyde de fer, de l'oxyde de cérium, de mélanges de ceux-ci et de mélanges de ceux-ci avec de l'oxyde d'aluminium et/ou du dioxyde de silicium.

12. Procédé de production de particules d'oxyde métallique revêtues selon la revendication 11, dans lequel l'oxyde métallique est le dioxyde de titane ou l'oxyde de zinc ou un mélange de ceux-ci ou un mélange de ceux-ci avec de l'oxyde d'aluminium et/ou de l'oxyde de fer et/ou du dioxyde de silicium.

13. Procédé de production de particules d'oxyde métallique revêtues selon l'une quelconque des revendications 1 à 12, dans lequel la taille des particules primaires des particules d'oxyde revêtues se situe dans la plage de 2 à 100 nm.

14. Particules d'oxyde métallique revêtues pouvant être obtenues selon le procédé de l'une quelconque des revendications 1 à 13.

15. Composition cosmétique ou dermatologique comprenant les particules d'oxyde métallique revêtues selon la revendication 14.

16. Composition cosmétique ou dermatologique selon la revendication 15, dans laquelle sont également présents d'autres agents d'écran contre les UV-A et/ou d'autres agents d'écran contre les UV-B et/ou d'autres agents d'écran à large bande.

17. Composition cosmétique ou dermatologique selon les revendications 15 ou 16, comprenant une quantité de 0,01 à 25 % en poids de particules d'oxyde métallique revêtues.

18. Utilisation des particules d'oxyde métallique revêtues telles que définies selon la revendication 14, pour la stabilisation de filtres UV.

19. Utilisation selon la revendication 18, dans laquelle le filtre UV est le dibenzoylméthane ou un dérivé de celui-ci.

20. Utilisation des particules d'oxyde métallique revêtues selon la revendication 14, pour protéger la peau ou les cheveux humains contre le rayonnement UV.
